(19) Europäisches Patentamt / European Patent Office / Office européen des brevets

(11) **EP 3 949 844 B1**

(12) **EUROPEAN PATENT SPECIFICATION**

(45) Date of publication and mention
of the grant of the patent:
**13.09.2023 Bulletin 2023/37**

(21) Application number: **19920877.8**

(22) Date of filing: **25.03.2019**

(51) International Patent Classification (IPC):
**A61B 5/287** (2021.01)　　**A61B 5/00** (2006.01)
**A61B 18/14** (2006.01)　　**A61M 25/00** (2006.01)

(52) Cooperative Patent Classification (CPC):
**A61M 25/005; A61B 5/287; A61B 5/6852;**
**A61B 18/1492;** A61B 2018/1467; A61M 25/0045

(86) International application number:
**PCT/JP2019/012621**

(87) International publication number:
**WO 2020/194474 (01.10.2020 Gazette 2020/40)**

(54) **CATHETER**

KATHETER

CATHÉTER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**09.02.2022 Bulletin 2022/06**

(73) Proprietor: **Japan Lifeline Co., Ltd.
Tokyo 140-0002 (JP)**

(72) Inventor: **SASAKI Takuya
Tokyo 140-0002 (JP)**

(74) Representative: **Grünecker Patent- und
Rechtsanwälte
PartG mbB
Leopoldstraße 4
80802 München (DE)**

(56) References cited:
**JP-A- 2016 137 019　　JP-A- 2016 137 019
JP-A- 2017 148 472　　US-A- 5 057 092
US-A1- 2011 251 519　　US-A1- 2013 338 467
US-A1- 2018 207 399　　US-A1- 2018 360 333
US-B1- 6 477 396**

## Description

Technical Field

The present invention relates to a catheter comprising a catheter shaft reinforced by a braid in which a metal element wire or a metal element-wire bundle is braided.

Background Art

Hitherto, an electrode catheter in which a proximal end portion of a catheter shaft is constituted by a resin tube (braided tube) reinforced by a metal braid, such as stainless steel braid, has been known (refer to, for example, Patent Literature 1 below).

By constituting the proximal end portion of the catheter shaft by a braided tube, it is possible to improve to a certain degree the torque transmission properties of the electrode catheter.

However, even the electrode catheter comprising the catheter shaft in which the proximal end portion is constituted by a braided tube is not one having sufficient torque transmission properties, and further improvement is desired.

In particular, in an electrode catheter that is inserted into a body by using a guide wire, since a large-diameter center lumen is formed as a guide wire lumen in a catheter shaft that constitutes the electrode catheter, the proportion that the space occupies in the shaft is high (the proportion of resin that constitutes the shaft is low), and sufficient torque transmission properties cannot be exhibited, as a result of which the improvement of the torque transmission properties in the electrode catheter that has the guide wire lumen is an important issue.

With regard to such an issue, the present inventor and others propose an electrode catheter comprising a catheter shaft that is made up of a braided tube reinforced by braids over the entire length, and that is a braided tube in which at least the braid that reinforces a distal end portion is made of resin (refer to Patent Literature 2 below).

Specifically, there are proposed an electrode catheter comprising a catheter shaft that is made up of a braided tube reinforced by a resin braid over its entire length (Claim 2 of Patent Literature 2) and an electrode catheter comprising a catheter shaft that has a distal end portion reinforced by a resin braid and a proximal end portion reinforced by a metal braid (Claim 3 of Patent Literature 2). Further relevant prior art is described in JP 2016 137019 A, US 2011/251519 A1, US 2013/338467 A1, US 2018/207399 A1.

Citation List

Patent Literature

PTL 1: Japanese Unexamined Patent Application Publication No. 2013-123508 (one may also refer to US 2018/360333 A1)

PTL 2: Japanese Unexamined Patent Application Publication No. 2017-148472

Summary of Invention

Technical Problem

However, since the catheter comprising the catheter shaft reinforced by the resin braid over the entire length is such that the braid that reinforces the proximal end portion is also made of resin, the torque transmission properties cannot be sufficiently improved.

In addition, since the catheter comprising the catheter shaft having the distal end portion reinforced by the resin braid and the proximal end portion reinforced by the metal braid is manufactured by fusing a resin braided tube that constitutes the distal end portion and a metal braided tube that constitutes the proximal end portion, the resin braid that reinforces the distal end portion and the metal braid that reinforces the proximal end portion are not continuous, and thus the torque transmission properties cannot be sufficiently improved.

In addition, in the catheter that has such a structure, a kink tends to occur at a boundary between the distal end portion and the proximal end portion, at which the constituent materials of the braids change.

Accordingly, reinforcing not only the proximal end portion of the catheter shaft but also the distal end portion thereof by a metal braid can be considered.

However, when the distal end portion of the catheter shaft is reinforced by a metal braid, it becomes very difficult to process its tube wall to form a side hole for passing a lead wire of a ring-shaped electrode therethrough (to subject the tube wall to perforation processing involving cutting/removal of a metal element wire).

Even if a side hole is formed, a cut surface of the metal element wire that constitutes the braid is exposed at an inner peripheral surface of the side hole, and, when manufacturing and using the catheter, the cut surface of the metal element wire may extend into the side hole and come into contact with the lead wire of the ring-shaped electrode, and a resin covering layer that constitutes the lead wire may be damaged, as a result of which the insulation properties of the lead wire may be lost or the lead wire may break.

The present invention has been made based on such circumstances above. An object of the present invention is to provide a catheter that excels in torque transmission properties compared with conventional publicly known catheters, that makes it easy to process a tube wall at a distal end portion of a catheter shaft to form a side hole for passing a lead wire of a ring-shaped electrode therethrough, and that does not allow a resin covering layer of the lead wire that is inserted through the side hole to peel or the lead wire to break.

Solution to Problem

In particular, a catheter is provided having the features defined in claim 1. Further preferred embodiments are defined in the dependent claims. A catheter of the present invention comprises a catheter shaft that has at least one lumen, at least one ring-shaped electrode that is mounted at an outer peripheral surface of a distal end portion of the catheter shaft, and a lead wire that is made up of a metal core wire covered with a resin and that is connected to the ring-shaped electrode,

wherein the catheter shaft is a braided tube that is made up of a braid that is embedded in a tube wall over an entire length of the catheter shaft, the braid being formed from a metal element wire or a metal element-wire bundle,
wherein a side hole that extends from the outer peripheral surface to the lumen is formed in the tube wall at the distal end portion of the catheter shaft in correspondence with a mounting position of the ring-shaped electrode without cutting the metal element wire or the metal element-wire bundle,
wherein the lead wire is connected to the ring-shaped electrode by joining a distal end portion of the lead wire to an inner peripheral surface of the ring-shaped electrode, and the lead wire is inserted into the side hole and thus passes through a mesh of the braid, enters the lumen of the catheter shaft, and extends in the lumen,
wherein the side hole in which the lead wire is inserted is filled with a resin material, and
wherein, when a diameter of the lead wire is d and a diagonal length of the mesh of the braid in an axial direction of the catheter shaft is G, a G/d value is 1.2 or greater.

According to the catheter that has such a structure, since the catheter shaft is over its entire length reinforced by the braid formed from the metal element wire (bundle), the catheter excels in torque transmission properties compared with conventional publicly known catheters.

In addition, since the side hole that extends through the tube wall of the catheter shaft can be formed without cutting the metal element wire (bundle), it is possible to easily form the side hole.

Further, since the diagonal length (G) of the mesh of the braid is greater than or equal to 1.2 times the diameter (d) of the lead wire, even if the metal element wire (bundle) exists in the side hole, the lead wire can be passed through the mesh of the braid while preventing the lead wire from coming into contact with the metal element wire (bundle). Therefore, when manufacturing the catheter, it is possible to prevent the resin covering layer of the lead wire from peeling or the lead wire from breaking. In addition, since it is possible to maintain a non-contact state between the lead wire and the metal element wire (bundle) by filling the side hole in which the lead wire is inserted with the resin material, even when using the catheter (when the distal end portion of the catheter shaft is subjected to a bending operation), it is possible to prevent the resin covering layer of the lead wire from peeling or the lead wire from breaking.

(2) In the catheter of the present invention, it is preferable that the G/d value be 1.2 to 33.

Since the G/d value is 1.2 to 33, it is possible to prevent the resin covering layer of the lead wire from peeling or the lead wire from breaking, and to ensure a sufficient reinforcing effect by the braid.

(3) In the catheter of the present invention, it is preferable that, when an opening diameter of the side hole or an opening length of the side hole in the axial direction of the catheter shaft is P, and an electrode width of the ring-shaped electrode mounted at a position of formation of the side hole is W, Formulas (1) and (2) below hold. Here, "an opening diameter of the side hole" refers to the diameter of the opening of the side hole when the side hole is a round hole, and "an opening length of the side hole" refers to the opening length of the side hole in the axial direction of the shaft when the side hole is a long hole (slotted hole).

$$\text{Formula (1): } 1.0d \leq P \leq 0.9W$$

$$\text{Formula (2): } 0.1 \leq P/G \leq 4.0$$

According to the catheter that has such a structure, since the opening diameter (the opening length) P of the side hole is 1.0d or greater, it is possible to ensure the insertion of the lead wire into the side hole.

In addition, since the opening diameter (the opening length) P of the side hole is 0.9W or less, it is possible to reliably cover the opening of the side hole in the outer peripheral surface of the shaft with the ring-shaped electrode.

In addition, since the P/G value is 0.1 or greater, even if the metal element wire (bundle) exists in the side hole, the lead wire can be passed through the side hole while preventing the lead wire from coming into contact with the metal element wire (bundle).

In addition, since the P/G value is 4.0 or less, it is possible to easily perform the filling operation of filling the side hole with the resin material at the time of manufacture.

(4) In the catheter of the present invention, it is preferable that a diameter of the metal element wire (element wire circular in cross section) or a width of the metal element wire (element wire rectangular in cross section) or a width of the metal element-wire bundle be 0.03 to 0.1 mm, and at least a density of the braid at the distal end portion of the catheter shaft be 13 to 265 PPI (1 pick per cm corresponds to 2.54 picks per inch or PPI).

According to the catheter that has such a structure, since the braid is formed from the metal element wire (bundle) whose diameter or width is 0.03 to 0.1 mm, and the density of the braid at the distal end portion of the catheter shaft is 13 PPI or greater, it is possible to exhibit a sufficient reinforcing effect by the braid and thus effect of improving the torque transmission properties.

In addition, since the braid is formed from the metal element wire (bundle) whose diameter or width is 0.03 to 0.1 mm, and the density of the braid at the distal end portion of the catheter shaft is 265 PPI or less, it is possible for the diagonal length (G) of the mesh of the braid to be sufficiently long, and to ensure a value of 1.2 or greater for the G/d value (to pass the lead wire through the mesh of the braid).

Advantageous Effects of Invention

The catheter of the present invention excels in torque transmission properties compared with conventional publicly known catheters, makes it easy to, at the time of manufacture, form a side hole for passing the lead wire of the ring-shaped electrode therethrough in the tube wall at the distal end portion of the catheter shaft, and, at the time of manufacture and use, does not allow the resin covering layer of the lead wire to peel or the lead wire to break.

Brief Description of Drawings

[Fig. 1] Fig. 1 is a side view showing an electrode catheter according to a first embodiment of the present invention.

[Fig. 2] Fig. 2 is a side view showing a catheter shaft that constitutes the electrode catheter shown in Fig. 1.

[Fig. 3A] Fig. 3A is a cross sectional view (IIIA-IIIA sectional view) of the catheter shaft shown in Fig. 2.

[Fig. 3B] Fig. 3B is a cross sectional view (IIIB-IIIB sectional view) of the catheter shaft shown in Fig. 2.

[Fig. 3C] Fig. 3C is a cross sectional view (IIIC-IIIC section view) of the catheter shaft shown in Fig. 2.

[Fig. 4] Fig. 4 is a sectional view schematically showing a form in which, in the electrode catheter shown in Fig. 1, a lead wire of a ring-shaped electrode enters a lumen of the catheter shaft by passing through a side hole.

[Fig. 5] Fig. 5 is a sectional view showing a state of connection between an inner tube and a distal end tip that constitute the catheter shaft shown in Fig. 2.

[Fig. 6] Fig. 6 is an explanatory view schematically showing a main portion of the electrode catheter shown in Fig. 1.

Description of Embodiments

<Embodiment>

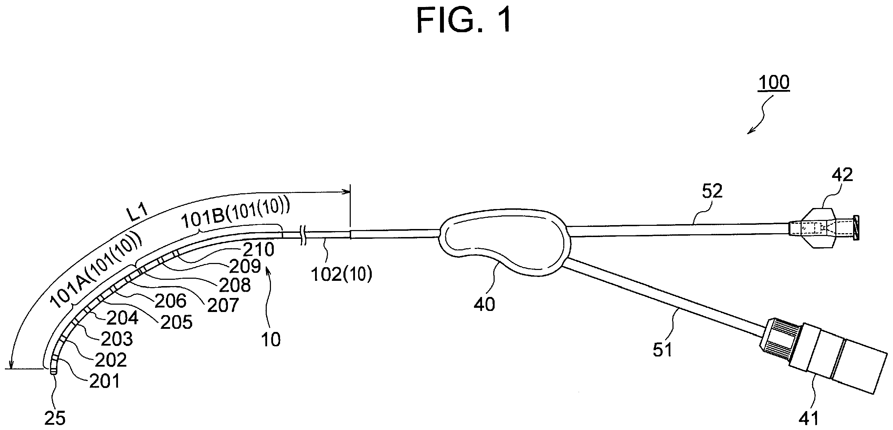

An electrode catheter 100 of this embodiment shown in Figs. 1 to 6 is one that is used for measuring an electrical potential of a part such as a coronary artery of the heart.

The electrode catheter 100 comprises a catheter shaft 10, ring-shaped electrodes 201 to 210 that are mounted at an outer peripheral surface of a distal end portion 101 of the catheter shaft 10, and lead wires 301 to 310 that are connected to each of the ring-shaped electrodes 201 to 210; the catheter shaft 10 is constituted by an inner tube 11 that has a guide wire lumen 11L and an outer tube 13 which forms, with an outer peripheral surface of the inner tube 11, a lumen 12L for inserting the lead wires 301 to 310; a braid 183 formed from metal element-wire bundles 181 is embedded in a tube wall of the outer tube 13 along the entire length of the catheter shaft 10; side holes 15 that extend from the outer peripheral surface to the lumen 12L are formed in the tube wall of the outer tube 13 at the distal end portion 101 of the catheter shaft 10 in correspondence with mounting positions of the ring-shaped electrodes 201 to 210 without cutting the metal element-wire bundle 181; the lead wires 301 to 310 are electrically connected to the ring-shaped electrodes 201 to 210 by joining a distal end portion of each of the lead wires 301 to 310 to an inner peripheral surface of each of the ring-shaped electrodes 201 to 210, and each of the lead wires 301 to 310 is inserted into the side holes 15 provided in correspondence with the mounting positions of the ring-shaped electrodes 201 to 210 and thus passes through meshes of the braid 183, enters the lumen 12L from the side holes 15, and extends to the lumen 12L; and the side holes 15 in which each of the lead wires 301 to 310 is inserted are filled with a resin material (an adhesive) 16; and, when the diameter of each of the lead wires 301 to 310 is d and the diagonal length of the meshes of the braid 183 in an axial direction of the catheter shaft 10 is G, a G/d value is 1.2 to 33.

In Fig. 1, 25 denotes a distal end tip mounted on a distal end side of the catheter shaft 10, 40 denotes a handle connected to a proximal end side of the catheter shaft 10, 41 denotes a connector having incorporated therein a plurality of terminals to which proximal end portions of each of the lead wires 301 to 310 are connected, 42 denotes a guide wire port, 51 denotes a lead-wire protective tube having a single lumen structure that contains the lead wires 301 to 310 extending out from the handle 40 toward the connector 41, and 52 denotes a guide-wire protective tube having a single lumen structure that contains the inner tube 11 extending out from the handle 40 toward the guide wire port 42.

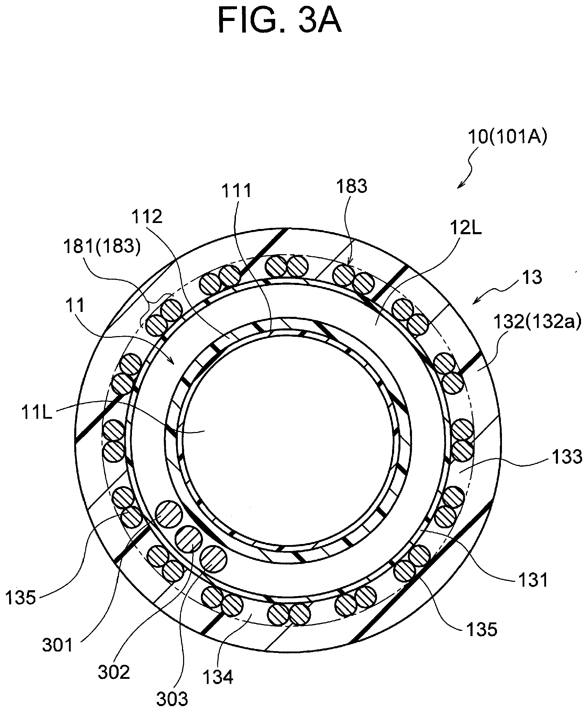

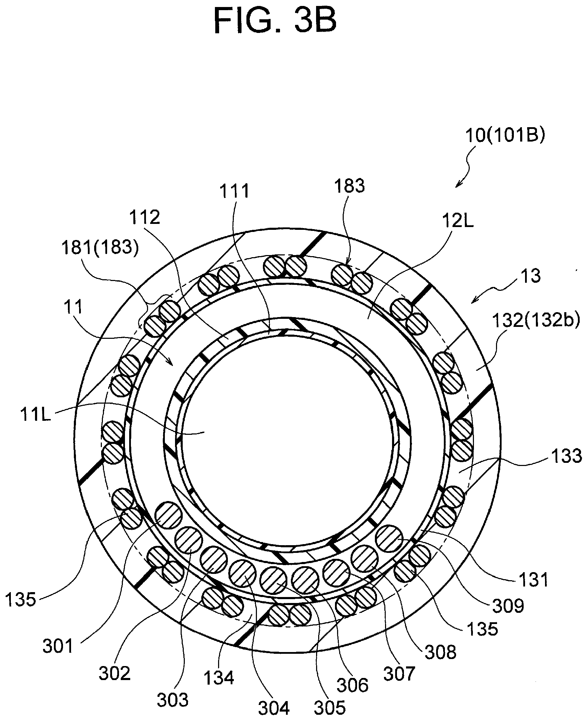

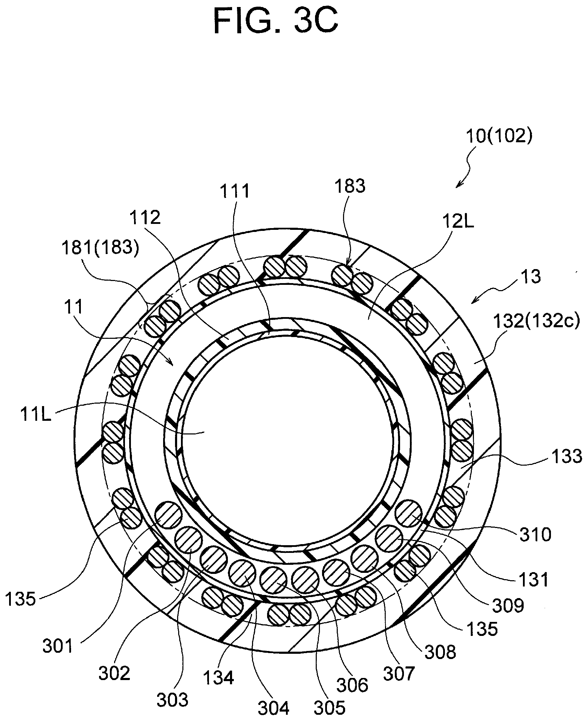

As shown in Figs. 3A to 3C, the catheter shaft 10 that constitutes the electrode catheter 100 is a double-tube-structure shaft that is constituted by the inner tube 11 and the outer tube 13.

In addition, the catheter shaft 10 is made up of the distal end portion 101 and a proximal end portion 102, and the distal end portion 101 is made up of a distal-end-side low-hardness region 101A and a hardness gradient region 101B.

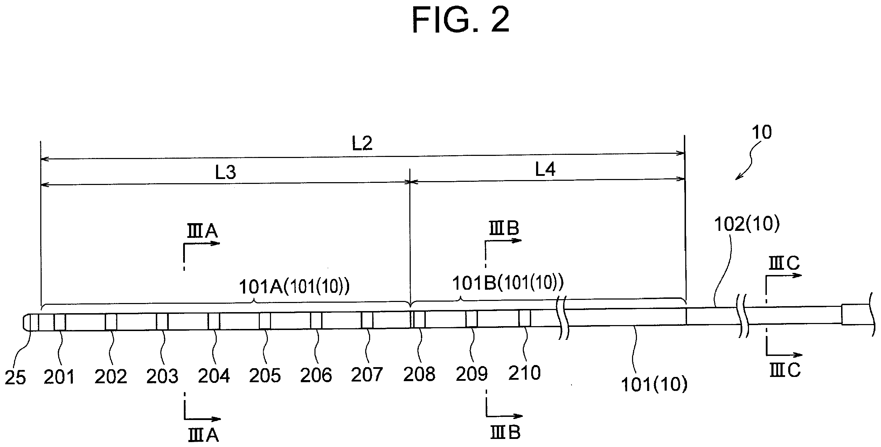

As shown in Fig. 1, the distal end portion 101 of the catheter shaft 10 has a specific curve shape. Note that, in Fig. 2, the distal end portion 101 that actually has a curve shape is shown as having a linear shape.

Although the distal end portion 101 that has a specific curve shape (that is stored) is easily deformed by being subjected to an external force (for example, by inserting the catheter shaft 10 into a tube), when the external force is removed, the distal end portion 101 can be restored to the stored curve shape.

An effective length (L1) of the catheter shaft 10 is ordinarily 400 to 1500 mm, with a preferred example being 650 mm.

In addition, a length (L2) of the distal end portion 101 is ordinarily 50 to 200 mm, with a preferred example being 95 mm.

In addition, a length (L3) of the distal-end-side low-hardness region 101A of the distal end portion 101 is ordinarily 20 to 100 mm, with a preferred example being 45 mm.

In addition, a length (L4) of the hardness gradient region 101B of the distal end portion 101 is ordinarily 20 to 100 mm, with a preferred example being 50 mm.

The inner tube 11 that constitutes the catheter shaft 10 is a single lumen tube having a two-layer structure made up of an inner layer 111 and an outer layer 112, and the guide wire lumen 11L is formed by the inner tube 11.

Here, as a constituent material of the inner layer 111, for example, high-density polyethylene can be used. In addition, as a constituent material of the outer layer 112, for example, polyether block amide (PEBAX) can be used.

The inside diameter of the inner tube 11 is ordinarily 0.5 to 1.5 mm, with a preferred example being 1.0 mm.

The outside diameter of the inner tube 11 is ordinarily 0.6 to 1.7 mm, with a preferred example being 1.2 mm.

The braid 183 formed from the metal element-wire bundles 181 is embedded as a reinforcing material in the tube wall of the outer tube 13 that constitutes the catheter shaft 10 over the entire length of the catheter shaft 10 (the distal end portion 101 and the proximal end portion 102) .

That is, the outer tube 13 is made up of a braided tube, which is a resin tube, reinforced by the metal braid 183.

As shown in Figs. 3A to 3C, the outer tube 13 is a single lumen tube having a three-layer structure made up of an inner layer 131, a reinforcing layer 133 provided by the braid 183, and an outer layer 132, and the lumen 12L is formed by an inner peripheral surface of the outer tube 13 and the outer peripheral surface of the inner tube 11.

As a constituent material (inside surface resin) of the inner layer 131 of the outer tube 13, synthetic resin, such as polyolefin, polyamide, polyether polyamide, polyurethane, nylon, or polyether block amide (PEBAX), can be used, among which PEBAX is preferably used.

Here, the constituent material of the inner layer 131 has the same hardness over the entire length of the catheter shaft 10. The hardness of the constituent material of the inner layer 131 is, for example, 72D.

The wall thickness of the inner layer 131 is ordinarily 10 to 50 $\mu$m, with a preferred example being 25 $\mu$m.

In addition, as a constituent material (outer surface resin) of the outer layer 132 of the outer tube 13, resins whose types are the same as the types of the constituent resins of the inner layer 131 can be used, among which PEBAX is preferably used.

The hardness of the constituent material of the outer layer 132 varies along a length direction of the catheter shaft 10. For example, the hardness of the outer layer 132 (132a) that constitutes the distal-end-side low-hardness region 101A of the distal end portion 101 is 35D, and the hardness of the outer layer 132 (132c) that constitutes the proximal end portion 102 is 72D. The hardness of the outer layer 132 (132b) that constitutes the hardness gradient region 101B of the distal end portion 101 changes with a gradient (is reduced) from 72D up to 35D toward a distal end direction.

The wall thickness of the outer layer 132 is ordinarily 30 to 200 $\mu$m, with a preferred example being 95 $\mu$m.

The reinforcing layer 133 of the outer tube 13 is made up of the metal braid 183 and resin 134 with which gaps in the metal element-wire bundles 181 that constitute the braid 183 are filled.

The number of carriers of the braid 183 is 16 and the number of ends of the braid 183 is 2, and, in sectional view of Figs. 3A to 3C, sixteen metal element-wire bundles 181, each being made up of two metal element wires, are disposed at equiangular (22.5°) intervals in a circumferential direction.

Note that the number of carriers and the number of ends of the braid that reinforces the outer tube 13 can be changed as appropriate. Here, it is preferable that the number of carriers be 8 to 32, and the number of ends of the braid be 1 to 4.

The resin 134 that constitutes the reinforcing layer 133 is one that flows into and fills a gap in a resin wire rod due to a portion of the constituent material of the outer layer 132 being fused when fabricating the outer tube 13.

The constituent material of the braid 183 (the metal element-wire bundles 181) is not particularly limited, and all publicly known metal materials that constitute braided tubes can be used, and, for example, stainless steel or the like can be an exemplification.

The wall thickness of the reinforcing layer 133 is ordinarily 60 to 200 $\mu$m, with a preferred example being 120 $\mu$m.

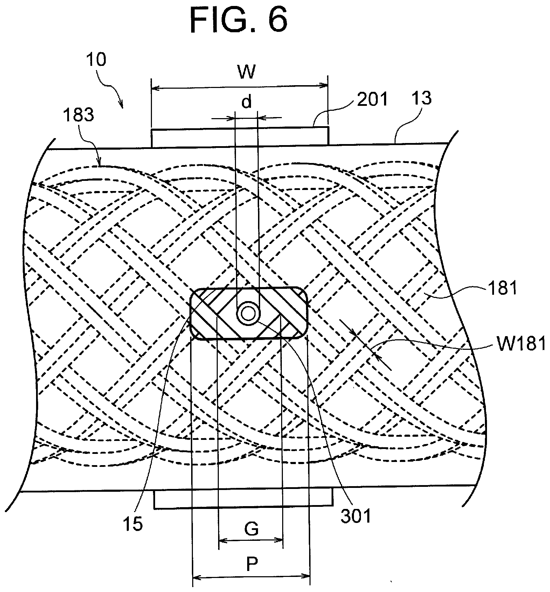

It is preferable that the width of the metal element-wire bundles 181 that constitute the braid 183 (W181 shown in Fig. 6) be 0.04 to 0.2 mm, with a preferred example being 0.12 mm.

In addition, the wire diameter of the metal element wires that constitute the metal element-wire bundles 181 (W181/number of ends) is ordinarily 0.02 to 0.1 mm, with a preferred example being 0.06 mm (0.12 mm/2).

The inside diameter of the outer tube 13 is ordinarily 0.7 to 2.0 mm, with a preferred example being 1.5 mm.

The outside diameter of the outer tube 13 is ordinarily 1.3 to 3.0 mm, with a preferred example being 2.0 mm.

The outer tube 13, which is a braided tube, can be manufactured by forming the braid 183 on an outer peripheral surface of a tubular inner-layer formation material, disposing an outer-layer formation material on an outer peripheral surface of the braid 183, and heating a tubular layered body obtained in this way at a temperature greater than or equal to the melting point of the inner-layer formation material and the outer-layer formation material.

As shown in Figs. 1 and 2, the ring-shaped electrodes 201 to 210 that constitute the electrode catheter 100 are mounted at the outer peripheral surface of the distal end portion 101 (the distal-end-side low-hardness region 101A and the hardness gradient region 101B) of the catheter shaft 10. Distal end portions of the lead wires 301 to 310 are joined to respective inner peripheral surfaces of the ring-shaped electrodes 201 to 210.

As a constituent material of the ring-shaped electrodes 201 to 210, for example, a metal having good electrical conductivity, such as platinum, gold, silver, aluminum, copper, or stainless steel can be used. Note that from the viewpoint of providing good radiopacity with respect to X rays, for example, platinum, gold, silver, and alloys containing any of these metals as main components are preferable.

It is preferable that the width of the ring-shaped electrodes 201 to 210 (W shown in Fig. 6. Length in the axial direction of the catheter shaft 10) be 0.5 to 10 mm, with a preferred example being 1 mm.

In addition, the distance between the electrodes, that is, the ring-shaped electrodes 201 to 210 is, for example, 1 to 10 mm.

Ten side holes that extend from an outer peripheral surface of the outer tube 13 to the lumen 12L are formed in the tube wall of the outer tube 13 at the distal end portion 101 of the catheter shaft 10 in correspondence with the mounting positions of the ring-shaped electrodes 201 to 210 without cutting the metal element-wire bundles 181 (the metal element-wire bundles 181 that pass through locations at which the side holes 15 should be formed).

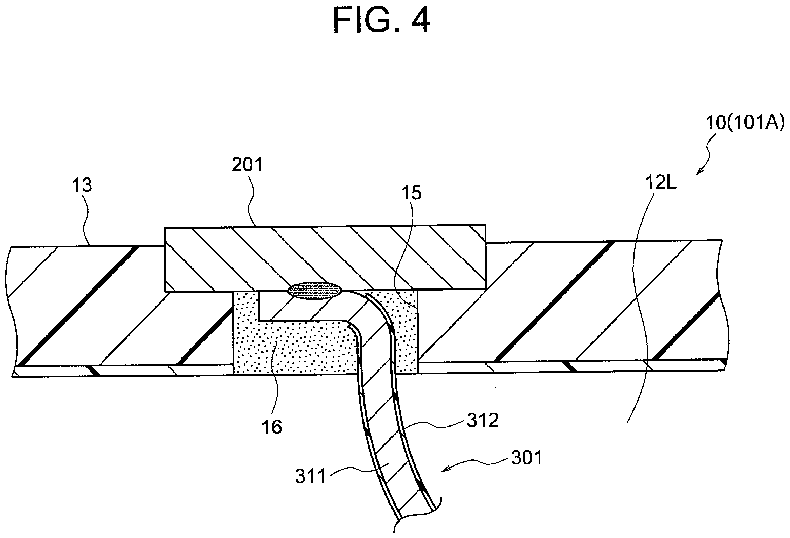

As shown in Fig. 4, the lead wire 301 whose distal end portion is joined to the inner peripheral surface of the ring-shaped electrode 201 passes through the mesh of the braid (not shown in Fig. 4) by being inserted into the side hole 15 formed in correspondence with the mounting position of the ring-shaped electrode 201, enters the lumen 12L from the side hole 15, and extends in the lumen 12L.

In Fig. 4, 311 denotes a metal core wire of the lead wire 301, 312 denotes a resin covering layer, and 16 denotes the resin material (the adhesive) with which the side hole 15 in which the lead wire 301 is inserted is filled.

Note that, in Fig. 4, although the braid (the metal element-wire bundles) that is embedded in the tube wall of the outer tube 13 is not shown, the lead wire 301 and the metal element-wire bundles are not in contact with each other.

In addition, the lead wires 302 to 310 that are joined to the respective inner peripheral surfaces of the ring-shaped electrodes 202 to 210 have the same form as that shown in Fig. 4.

It is preferable that the diameter (d) of the lead wires 301 to 310 be 0.04 to 0.15 mm, with a preferred example being 0.1 mm.

The distal end tip 25 is connected to the distal end side of the catheter shaft 10.

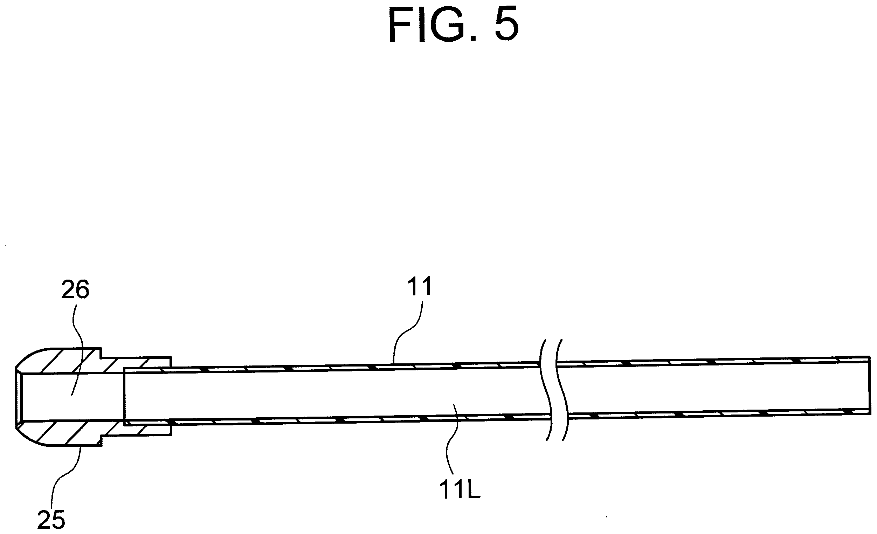

As shown in Fig. 5, a through hole 26 is formed in the distal end tip 25, and a distal end portion of the inner tube 11 is inserted in the through hole 26.

The catheter shaft 10 that has the double-tube structure is constituted by inserting the inner tube 11 to which the distal end tip 25 is connected in this way into the inside of the outer tube 13.

The proximal end portion of the catheter shaft 10 is inserted into the inside (an insertion path) of the handle 40. The insertion path of the catheter shaft 10 in the inside of the handle 40 branches off in two directions, and, in the inside of the handle 40, the lead wires 301 to 310 that extend to the lumen 12L of the catheter shaft 10, respectively, extend out from a proximal end of the outer tube 13 and extend along one of the branch paths, extend out from the handle 40, pass through a lumen of the lead-wire protective tube 51, and are inserted into the inside of the connector 41; and each of the proximal end portions of the lead wires 301 to 310 is connected to each of the plurality of terminals that are incorporated in the connector 41.

Therefore, the ring-shaped electrodes 201 to 210 are, respectively, electrically connected to each of the terminals in the connector 41.

On the other hand, in the inside of the handle 40, the inner tube 11 that constitutes the catheter shaft 10 extends out from the proximal end of the outer tube 13 and extends along the other branch path, extends out from the handle 40, and passes through a lumen of the guide-wire protective tube 52 and is connected to the guide wire port 42.

Therefore, a guide wire that is inserted from the guide wire port 42 can extend out from a distal end opening of the distal end tip 25 through the guide wire lumen 11L of the inner tube 11.

Fig. 6 is a schematic view for describing the relationship between the diameter (d) of the lead wire 301, the diagonal length (G) of the mesh of the braid 183 in the axial direction of the catheter shaft 10, the opening length (P) of the side hole 15 in the same direction, the width (W) of the ring-shaped electrode 201, and the width (W181) of the metal element-wire bundle 181 that constitutes the braid 183.

Note that the lead wires 302 to 310 that are joined to the respective inner peripheral surfaces of the ring-shaped electrodes 202 to 210 have the same form as that shown in Fig. 6.

As shown in Fig. 6, by braiding the metal element-wire bundle 181, the braid 183 having rhombus-shaped meshes are formed.

Here, it is preferable that the diagonal length (G) of the meshes of the braid 183 be 0.07 to 1.3 mm, with a preferred example being 0.3 mm.

In addition, the side hole 15 that has the form of a long hole (a slotted hole) is formed in the tube wall of the outer tube 13 in correspondence with the mounting position of the ring-shaped electrode 201 without cutting the metal element-wire bundle 181, and the metal element-wire bundle 181 exists in the side hole 15.

Here, it is preferable that the opening length (P) of the side hole 15 in the axial direction of the catheter shaft 10 be 0.04 to 9.9 mm, with a preferred example being 0.5 mm.

In the electrode catheter 100 of the embodiment, the value of the ratio (G/d) of the diagonal length (G) of the mesh with respect to the diameter (d) of the lead wire 301 is 1.2 or greater, and is preferably 1.2 to 33, with a preferred example being 5 (0.5 mm/0.1 mm).

Since the (G/d) value is 1.2 or greater, the lead wire 301 can be passed through the mesh of the braid 183 while preventing the lead wire 301 from coming into contact with the metal element-wire bundle 181.

When the value is less than 1.2, it is not possible or is very difficult to pass the lead wire through the mesh of the braid while not allowing the lead wire to come into contact with the metal element-wire bundle.

Since the (G/d) value is 33 or less, it is possible to ensure a sufficient reinforcing effect by the braid 183.

When this value is too large, it is not possible to exhibit a sufficient reinforcing effect with such a braid.

It is preferable that the opening length (P) of the side hole 15 be greater than or equal to 1.0 times the diameter (d) of the lead wire 301, be less than or equal to 0.9 times the width (W) of the ring-shaped electrode 201, and be 0.1 times to 4.0 times the diagonal length (G) of the mesh.

Since the opening length (P) of the side hole 15 is greater than or equal to 1.0 times the diameter (d) of the lead wire 301, it is possible to ensure the insertion of the lead wire 301 into the side hole 15.

Since the opening length (P) of the side hole 15 is less than or equal to 0.9 times the width (W) of the ring-shaped electrode 201, it is possible to reliably cover the opening of the side hole 15 with the ring-shaped electrode 201.

Since the opening length (P) of the side hole 15 is greater than or equal to 0.1 times the diagonal length (G) of the mesh, the lead wire 201 can be passed through the side hole 15 while preventing the lead wire 201 from coming into contact with the metal element-wire bundle 181 that exists in the side hole 15.

Since the opening length (P) of the side hole 15 is less than or equal to 4.0 times the diagonal length (G) of the mesh, it is possible to easily perform the filling operation of filling the side hole 15 with the resin material (the adhesive) 16 when manufacturing the electrode catheter 100.

It is preferable that the density of the braid 183 at the distal end portion of the catheter shaft 10 be 13 to 265 PPI (picks per inch), with a preferred example being 45 PPI (however, W181 = 0.06 mm).

With braids whose braid density is less than 13 (is low), it may not be possible to exhibit a sufficient reinforcing effect.

On the other hand, with braids whose braid density exceeds 265 (is high), it may not be possible for the diagonal length (G) of the mesh of the braid to be sufficiently long, and it may no longer be possible to ensure a value of 1.2 or greater for the G/d value (to pass the lead wire through the mesh of the braid).

According to the electrode catheter 100 of the present embodiment, since the catheter shaft 10 is over its entire length reinforced by the braid 183 formed by braiding the metal element-wire bundles 181, the catheter excels in torque transmission properties compared with conventional publicly known catheters.

In addition, since the side holes 15 at the distal end portion of the catheter shaft 100 can be formed without cutting the metal element-wire bundles 181 (for example, without using a high power laser device that is capable of cutting the metal element-wire bundles when forming the side holes 15), it is possible to easily form the side holes 15.

Further, since the diagonal length (G) of the meshes of the braid 183 is greater than or equal to 1.2 times the diameter (d) of the lead wires 301 to 310, each of the lead wires 301 to 310 can be passed through the meshes of the braid 183 while preventing each of the lead wires 301 to 310 from coming into contact with the metal element-wire bundles 181 that exist in the side holes 15.

Therefore, when manufacturing the electrode catheter 100, it is possible to prevent the resin covering layers of the lead wires 301 to 310 from peeling or the lead wires 301 to 310 from breaking. In addition, since it is possible to maintain a non-contact state between the lead wires 301 to 310 and the metal element-wire bundles 181 by filling the side holes 15 in which each of the lead wires 301 to 310 is inserted with the resin material 16, even when the electrode catheter 100 is used (when the distal end portion of the catheter shaft 10 is subjected to a bending operation), it is possible to prevent the resin covering layers of the lead wires 301 to 310 from peeling or the lead wires 301 to 310 from breaking.

Although one embodiment of the present invention has been described above, the present invention is not limited to these embodiments, and various changes are possible within the scope of the appended claims.

For example, the catheter of the present invention may be a defibrillation catheter comprising a first DC electrode group that are made up of a plurality of ring-shaped electrodes mounted at a distal end portion of a catheter shaft, a second DC electrode group that are made up of a plurality of ring-shaped electrodes spaced apart from the first DC electrode group toward a proximal end side and mounted at the distal end portion of the catheter shaft, a first DC lead wire group made up of lead wires connected to each of the electrodes that constitute the first DC electrode group, and a second DC lead wire group made up of lead wires connected to each of the electrodes that constitute the second DC electrode group.

In addition, the catheter of the present invention may be one in which the distal end portion of the catheter shaft can be subjected to a deflection operation, with the deflection direction being one direction (a single deflection type) or two directions (a bi-direction type).

In addition, although the catheter shaft of the embodiment above by being a double-tube structure has two lumens (the guide wire lumen 11L and the lumen 12L), the catheter shaft that constitutes the catheter of the present invention may be one made up of one multi-lumen tube having at least two lumens. In addition, a catheter shaft having a single-lumen structure can also constitute the catheter of the present invention.

Reference Signs List

100 electrode catheter

10 catheter shaft

101 distal end portion

101A distal-end-side low-hardness region

101B hardness gradient region

102 proximal end portion

11 inner tub

111 inner layer

112 outer layer

11L guide wire lumen

12L lumen

13 outer tube

131 inner layer

132 outer layer

133 reinforcing layer

134 resin

135 braid

15 side hole

16 resin material (adhesive)

181 metal element-wire bundle

183 braid

201 to 210 ring-shaped electrode

25 distal end tip

301 to 310 lead wire

40 handle

41 connector

42 guide wire port

51 lead-wire protective tube

52 guide-wire protective tube

**Claims**

1. A catheter (100) comprising a catheter shaft (10) that has at least one lumen (11L, 12L), at least one ring-shaped electrode (201 to 210) that is mounted at an outer peripheral surface of a distal end portion (101) of the catheter shaft (10), and a lead wire (301 to 310) that is made up of a metal core wire (311) covered with a resin (134) and that is connected to the ring-shaped electrode (201 to 210),

    wherein the catheter shaft (10) is a braided tube that is made up of a braid (135, 183) that is embedded in a tube wall over an entire length of the catheter shaft (10), the braid (135, 183) being formed from a metal element wire or a metal element-wire bundle (181),

wherein a side hole (15) that extends from the outer peripheral surface to the lumen (11L, 12L) is formed in the tube wall at the distal end portion (101) of the catheter shaft (10) in correspondence with a mounting position of the ring-shaped electrode (201 to 210) without cutting the metal element wire or the metal element-wire bundle (181),

wherein the lead wire (301 to 310) is connected to the ring-shaped electrode (201 to 210) by joining a distal end portion of the lead wire (301 to 310) to an inner peripheral surface of the ring-shaped electrode (201 to 210), and the lead wire (301 to 310) is inserted into the side hole (15) and thus passes through a mesh of the braid (135, 183), enters the lumen (11L, 12L) of the catheter shaft (10), and extends in the lumen (11L, 12L),

wherein the side hole (15) in which the lead wire (301 to 310) is inserted is filled with a resin material (16), and

wherein, when a diameter of the lead wire (301 to 310) is d and a diagonal length of the mesh of the braid (135, 183) in an axial direction of the catheter shaft (10) is G, a G/d value is 1.2 or greater.

2. The catheter (100) according to Claim 1, wherein the G/d value is 1.2 to 33.

3. The catheter (100) according to Claim 1 or Claim 2, wherein, when an opening diameter of the side hole (15) or an opening length of the side hole (15) in the axial direction of the catheter shaft (10) is P, and an electrode width of the ring-shaped electrode (201 to 210) mounted at a position of formation of the side hole (15) is W, Formulas (1) and (2) below hold:

$$\text{Formula (1): } 1.0d \leq P \leq 0.9W,$$

$$\text{Formula (2): } 0.1 \leq P/G \leq 4.0.$$

4. The catheter (100) according to any one of Claims 1 to 3, wherein a diameter or a width of the metal element wire or a width of the metal element-wire bundle (181) is 0.03 to 0.1 mm, and at least a density of the braid (135, 183) at the distal end portion (101) of the catheter shaft (10) is about 5.1 to 104 picks per cm PPC (13 to 265 PPI).

**Patentansprüche**

1. Katheter (100), umfassend einen Katheterschaft (10), der mindestens ein Lumen (11L, 12L), mindestens eine ringförmige Elektrode (201 bis 210), die an einer äußeren Umfangsoberfläche eines distalen Endabschnitts (101) des Katheterschafts (10) montiert ist, und einen Zuleitungsdraht (301 bis 310), der aus einem Metallkerndraht (311) besteht, der mit einem Harz (134) bedeckt ist und der mit der ringförmigen Elektrode (201 bis 210) verbunden ist, aufweist,

wobei der Katheterschaft (10) ein geflochtener Schlauch ist, der aus einem Geflecht (135, 183) besteht, das in einer Schlauchwand über eine gesamte Länge des Katheterschafts (10) eingebettet ist, wobei das Geflecht (135, 183) aus einem Metallelementdraht oder einem Metallelementdrahtbündel (181) ausgebildet ist,

wobei ein Seitenloch (15), das sich von der äußeren Umfangsoberfläche zu dem Lumen (11L, 12L) erstreckt, in der Schlauchwand an dem distalen Endabschnitt (101) des Katheterschafts (10) in Übereinstimmung mit einer Montageposition der ringförmigen Elektrode (201 bis 210) ausgebildet ist, ohne den Metallelementdraht oder das Metallelementdrahtbündel (181) zu schneiden,

wobei der Zuleitungsdraht (301 bis 310) mit der ringförmigen Elektrode (201 bis 210) durch Verknüpfen eines distalen Endabschnitts des Zuleitungsdrahts (301 bis 310) mit einer inneren Umfangsoberfläche der ringförmigen Elektrode (201 bis 210) verbunden ist, und der Zuleitungsdraht (301 bis 310) in das Seitenloch (15) eingeführt wird und somit durch ein Netz des Geflechts (135, 183) verläuft, in das Lumen (11L, 12L) des Katheterschafts (10) eintritt und sich in dem Lumen (11L, 12L) erstreckt,

wobei das Seitenloch (15), in das der Zuleitungsdraht (301 bis 310) eingeführt ist, mit einem Harzmaterial (16) gefüllt ist, und

wobei, wenn ein Durchmesser des Zuleitungsdrahtes (301 bis 310) d ist und eine diagonale Länge des Netzes des Geflechts (135, 183) in einer axialen Richtung des Katheterschafts (10) G ist, ein G/d-Wert 1,2 oder mehr beträgt.

2. Katheter (100) nach Anspruch 1, wobei der G/d-Wert 1,2 bis 33 beträgt.

3. Katheter (100) nach Anspruch 1 oder 2, wobei, wenn ein Öffnungsdurchmesser des Seitenlochs (15) oder eine Öffnungslänge des Seitenlochs (15) in der axialen Richtung des Katheterschafts (10) P ist und eine Elektrodenbreite der ringförmigen Elektrode (201 bis 210), die an einer Position der Ausbildung des Seitenlochs (15) montiert ist, W ist, die folgenden Formeln (1) und (2) gelten:

$$\text{Formel (1): } 1{,}0d \le P \le 0{,}9W,$$

$$\text{Formel (2): } 0{,}1 \le P/G \le 4{,}0.$$

4. Katheter (100) nach einem der Ansprüche 1 bis 3, wobei ein Durchmesser oder eine Breite des Metallelementdrahtes oder eine Breite des Metallelementdrahtbündels (181) 0,03 bis 0,1 mm beträgt und mindestens eine Dichte des Geflechts (135, 183) an dem distalen Endabschnitt (101) des Katheterschafts (10) etwa 5,1 bis 104 Fäden pro cm PPC (13 bis 265 PPI) beträgt.

## Revendications

1. Cathéter (100) comprenant une tige de cathéter (10) qui a au moins une lumière (11L, 12L), au moins une électrode en forme d'anneau (201 à 210) qui est montée au niveau d'une surface périphérique externe d'une partie d'extrémité distale (101) de la tige de cathéter (10), et un fil conducteur (301 à 310) qui se compose d'un fil d'âme métallique (311) recouvert d'une résine (134) et qui est relié à l'électrode en forme d'anneau (201 à 210),

   dans lequel la tige de cathéter (10) est un tube tressé qui se compose d'une tresse (135, 183) qui est incorporée dans une paroi de tube sur toute une longueur de la tige de cathéter (10), la tresse (135, 183) étant formée d'un fil d'élément métallique ou d'un faisceau de fils d'élément métalliques (181),
   dans lequel un trou latéral (15) qui s'étend de la surface périphérique externe jusqu'à la lumière (11L, 12L) est formé dans la paroi de tube au niveau de la partie d'extrémité distale (101) de la tige de cathéter (10) en correspondance avec une position de montage de l'électrode en forme d'anneau (201 à 210) sans couper le fil d'élément métallique ou le faisceau de fils d'élément métalliques (181),
   dans lequel le fil conducteur (301 à 310) est relié à l'électrode en forme d'anneau (201 à 210) en joignant une partie d'extrémité distale du fil conducteur (301 à 310) à une surface périphérique interne de l'électrode en forme d'anneau (201 à 210), et le fil conducteur (301 à 310) est inséré dans le trou latéral (15) et passe ainsi à travers une maille de la tresse (135, 183), entre dans la lumière (11L, 12L) de la tige de cathéter (10), et s'étend dans la lumière (11L, 12L),
   dans lequel le trou latéral (15) dans lequel le fil conducteur (301 à 310) est inséré est rempli d'un matériau en résine (16), et
   dans lequel, lorsqu'un diamètre du fil conducteur (301 à 310) est d et une longueur diagonale de la maille de la tresse (135, 183) dans une direction axiale de la tige de cathéter (10) est G, une valeur G/d est de 1,2 ou supérieure.

2. Cathéter (100) selon la revendication 1, dans lequel la valeur G/d est de 1,2 à 33.

3. Cathéter (100) selon la revendication 1 ou la revendication 2, dans lequel, lorsqu'un diamètre d'ouverture du trou latéral (15) ou une longueur d'ouverture du trou latéral (15) dans la direction axiale de la tige de cathéter (10) est P, et qu'une largeur d'électrode de l'électrode en forme d'anneau (201 à 210) montée au niveau d'une position de formation du trou latéral (15) est W, les formules (1) et (2) ci-dessous sont maintenues :

$$\text{Formule (1) : } 1{,}0\, d \le P \le 0{,}9\, W,$$

$$\text{Formule (2) : } 0{,}1 \le P/G \le 4{,}0.$$

4. Cathéter (100) selon l'une quelconque des revendications 1 à 3, dans lequel un diamètre ou une largeur du fil d'élément métallique ou une largeur du faisceau de fils d'élément métalliques (181) est de 0,03 à 0,1 mm, et au moins une masse volumique de la tresse (135, 183) au niveau de la partie d'extrémité distale (101) de la tige de cathéter (10) est d'environ 5,1 à 104 duites par cm PPC (13 à 265 PPI).

# FIG. 1

# FIG. 2

EP 3 949 844 B1

# FIG. 3A

# FIG. 3B

# FIG. 3C

FIG. 4

# FIG. 5

# FIG. 6

EP 3 949 844 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016137019 A
- US 2011251519 A1
- US 2013338467 A1
- US 2018207399 A1
- JP 2013123508 A
- US 2018360333 A1
- JP 2017148472 A